# EUROPÄISCHE PATENTSCHRIFT

(11) **EP 1 182 997 B1**
(45) Veröffentlichungstag und Bekanntmachung des Hinweises auf die Patenterteilung: **24.11.2004**
(21) Anmeldenummer: 00940327.0
(22) Anmeldetag: 06.06.2000
(51) Int. Cl.: A61F 9/01

(54) **VORRICHTUNG FÜR EINE MEDIZINISCHE BEHANDLUNG DES AUGES MIT LASERSTRAHLUNG**
DEVICE FOR MEDICAL TREATMENT OF THE EYES USING LASER ILLUMINATION
DISPOSITIF POUR EFFECTUER UN TRAITEMENT MEDICAL DE L'OEIL PAR FAISCEAU LASER

(30) Priorität: 10.06.1999 DE 19926476
(43) Veröffentlichungstag der Anmeldung: 06.03.2002
(73) Patentinhaber: WaveLight Laser Technologie AG, 91058 Erlangen (DE)
(72) Erfinder: DONITZKY, Christof, D-90542 Eckental (DE); LÖFFLER, Joachim, D-90562 Heroldsberg (DE)
(74) Vertreter: von Hellfeld, Axel, Dr. Dipl.-Phys.
(86) Internationale Anmeldenummer: PCT/EP2000/005197
(87) Internationale Veröffentlichungsnummer: WO 2000/076435

(56) Entgegenhaltungen:
- EP-A- 0 765 648
- WO-A-99/20174
- DE-U- 29 809 759
- US-A- 4 848 340

## Beschreibung

Die Erfindung betrifft eine Vorrichtung für eine medizinische Behandlung und/oder Operation des Auges mit Laserstrahlung unter Verwendung von Hilfsstrahlung zur Ermittlung der Augenstellung.

Insbesondere kann eine solche Vorrichtung für die sogenannte PRK (Photo-Refraktive Keratektomie, englisch: photo refractive keratectomie) verwendet werden, d. h. ein Verfahren zur Korrektur der Fehlsichtigkeit des menschlichen Auges, bei dem insbesondere die Cornea neu geformt wird. Besonders eignet sich die erfindungsgemäße Vorrichtung für das sogenannte LASIK-Verfahren, bei dem zunächst ein kleiner Lappen (Deckel) aus Epithel, Bowman-Membran und Stroma geschnitten und aufgeklappt wird und sodann die PRK im Stromabett durchgeführt wird.

Es ist bekannt, bei der Laserbehandlung des menschlichen Auges unterschiedliche Laser einzusetzen, im Zusammenhang mit der vorliegenden Erfindung sind dies insbesondere Excimerlaser (mit einer Wellenlänge von z. B. 193 nm) oder auch Er:YAG-Festkörperlaser.

Bei der PRK (insbesondere LASIK) wird Material der Hornhaut abgetragen. Der Abtrag ist eine Funktion der auf die Hornhaut auftreffenden Energiedichte (Energie pro Flächeneinheit) des Laserstrahls. Es sind unterschiedliche Techniken für die Strahlformung und Strahlführung bekannt, so zum Beispiel die sogenannte Schlitz-Abtastung (slit scanning), bei der die Strahlung mittels eines bewegten Schlitzes über den zu bearbeitenden Bereich geführt wird, das sogenannte Fleck-Abtasten (spot-scanning), bei dem ein Strahlungsfleck mit sehr geringen Abmessungen über das abzutragende Gebiet geführt wird, und auch die sogenannte Vollabtragung (full-ablation), bei der die Strahlung großflächig über den gesamten abzutragenden Bereich eingestrahlt wird und wobei die Energiedichte sich über das Strahlprofil ändert, um den gewünschten Abtrag der Hornhaut zu erreichen. Der Stand der Technik kennt für die genannten Strahl-Führungen jeweils geeignete Algorithmen zum Steuern der Strahlung, um die Hornhautoberfläche so abzutragen, daß die Cornea schließlich den gewünschten Krümmungsradius erhält.

Das vorstehend bereits erwähnte "Fleck-Abtasten" (spot-scanning) verwendet einen auf einen relativ kleinen Durchmesser (0,1-2mm) fokussierten Laserstrahl, der mittels einer Strahlführungseinrichtung auf verschiedene Stellen der Hornhaut gerichtet und durch einen sogenannten Abtaster (scanner) sukzessive so bewegt wird, daß letztlich der gewünschte Abtrag von der Cornea erreicht wird. Bei der PRK sind insbesondere sogenannte galvanometrische Abtaster (Scanner) verwendbar (vgl. Aufsatz G.F. Marshall in LASER FOCUS WORLD, Juni 1994, S. 57). Die vorliegende Erfindung betrifft insbesondere das sog. spot-scanning beim LASIK-Verfahren.

Ein besonderes Problem bei der PRK und LASIK ist die relative Positionierung von Laserstrahl und Auge. Aus medizinischen Gründen ist eine mechanische Fixierung des Auges nicht befriedigend. Der Stand der Technik kennt deshalb eine sogenannte optische Fixierung, bei der mit dem materialbearbeitenden Laserstrahl in der Regel koaxial ein sogenannter Fixierstrahl verwendet wird. Der Patient ist angehalten, genau auf den durch den Fixierstrahl definierten Punkt zu schauen, damit das Auge während der gesamten Operation immer die gleiche Position einnimmt. Allerdings gelingt dies nicht, jedenfalls nicht mit hinreichender Zuverlässigkeit, so daß es zu Bewegungen des Auges kommt, die den gesamten Ablationsvorgang massiv beeinträchtigen können.

Der Stand der Technik kennt sog. "eye-tracker"; dies sind Einrichtungen, die Bewegungen des Auges ermitteln, um dann den für die Ablation verwendeten Laserstrahl entsprechend den Augenbewegungen zu steuern (nachzuführen). Zum Stand der Technik wird auf folgende Dokumente hingewiesen:

Gobbi, Pier Giorgie et al.: Automatic Eye Tracker for Excimer Laser Photorefractive Keratectomy; Supplement to Journal of Refractive Surgery, Vol. 11, Mai/Juni 1995; weiterhin: Lin, J.T., Ophtalmic Surgery Method Using Non-Contact Scanning Laser, U.S. Patent 5,520,679, 28. Mai 1996; und Manns, Fabrice, et al., Optical profilometry of poly(methylmethacrylate) surfaces after reshaping with a scanning photorefractive keratectomy (SPRK) system, Zeitschrift APPLIED OPTICS, Vol. 35. NO. 19, 1. Juli 1996.

Weiterhin wird verwiesen auf das deutsche Gebrauchsmuster 298 09 759.1. Dort wird für die Hilfsstrahlung, welche der Ermittlung der Augenstellung für das "eye-tracking" dient, weißes Licht verwendet, das von Leuchtdioden emittiert wird.

Die DE 197 02 335 Cl beschreibt ein Lasersystem zur Behandlung der Cornea mit Einrichtungen, um den Laser-Behandlungsstrahl dann, wenn sich das Auge relativ zu einer Bezugsachse bewegt, nachzuführen. Hierzu wird eine Bildaufnahmeeinrichtung (CCD-Kamera) verwendet und es wird Hilfsstrahlung eingesetzt, um das Auge für die Erzeugung der Bildaufnahmen zu beleuchten. Entsprechend einer Bewegung des Auges relativ zur Bezugsachse steuert eine Steuerung eine Strahlführungseinrichtung, z. B. einen galvanometrischen Abtaster. Für die Bildfolgefrequenz, mit der die Kamera Bilder aufnimmt, wird eine bestimmte Abhängigkeit in Bezug auf die Pulsfolgefrequenz der Laserstrahlung vorgeschlagen. Dieser Stand der Technik wird hier als bekannt vorausgesetzt und benutzt. Zusammengefaßt lehrt er, in schneller Folge Bilder des Auges mit einer Kamera aufzunehmen und zu verarbeiten, um Bewegungen des Auges zu ermitteln. Aus aufeinanderfolgenden Bildern (z.B. zwei aufeinanderfolgenden Bildern) kann eine Veränderung der Position des Auges (Pupillenlage) ermittelt werden. Entsprechend der Augenbewegung läßt sich dann der Ablations-Laserstrahl mittels geeigneter Strahlführungseinrichtungen (z.B. dem genannten galvanometrischen Scanner) nachführen.

Eine Vorrichtung gemäß dem Oberbegriff des Anspruchs 1 geht aus der WO 99/20174 hervor.

Bei der PRK (insbesondere LASIK) werden somit zumindest drei unterschiedliche Strahlungen unterschieden. Zum einen der eigentliche Behandlungslaserstrahl, der die Ablation bewirkt, zum anderen die sog. Hilfsstrahlung, also diejenige Strahlung, die der Beleuchtung des Auges dient, um z. B. mit der Kamera die Augenposition festzustellen und zum Dritten gegebenenfalls der sog. Fixierstrahl, der ortsfest ist und den Patienten veranlassen soll, mit dem Auge immer den gleichen Punkt anzuschauen (letzteres ist nur eine besondere Option).

Im Stand der Technik werden zur Ausleuchtung des Auges Halogenleuchten verwendet, die mittels eines Faserbündels in den Beobachtungsstrahlengang gebracht werden. Stand der Technik ist auch die Verwendung einer Ringleuchte oder die koaxiale Einkopplung der Beleuchtungsstrahlung über ein Operationsmikroskop. Bekannt sind auch flexible Schwanenhälse für die Positionierung des Beleuchtungslichtes zum Ausleuchten des Augenvordergrundes, insbesondere der Hornhaut so, daß der Arzt wahlweise sich das Licht für eine optimale Beobachtung des Auges als Ganzes einstellen kann. Die im Stand der Technik verwendeten Halogenglühlampen und Xenonlampen sind sowohl hinsichtlich der Belastung des Patienten als auch hinsichtlich der Beleuchtungsqualität für den behandelnden Arzt verbesserungsfähig.

Wird insbesondere beim spot-scanning-Verfahren für LASIK zur Ermittlung der Augenstellung die Iris und die Pupille mit einem Kamerasystem aufgenommen und anschließend (on-line) der Pupillenschwerpunkt berechnet, so ist für die optimale Erkennung der Pupille ein hoher Kontrast zwischen Pupille und Iris erforderlich. Es hat sich gezeigt, daß die Hilfsstrahlung hinsichtlich ihrer Einstrahlwinkel, Wellenlängen etc. äußerst wichtig ist für die Erzielung guter Ergebnisse bei der Ermittlung der Augenstellung.

Dementsprechend ist es Aufgabe der vorliegenden Erfindung, eine Vorrichtung für die medizinische Behandlung des Auges der eingangs genannten Art so auszugestalten, daß bei der Ermittlung der Augenstellung zuverlässige Ergebnisse erreicht werden, insbesondere dann, wenn das System bei unterschiedlichen Augen (Patienten) und unter unterschiedlichen Bedingungen eingesetzt wird.

Erfindungsgemäße Lösungen dieser Aufgabe sind in den Patentansprüchen beschrieben.

Der Erfindung liegt die Erkenntnis zugrunde, daß besonders gute Meßergebnisse bezüglich der Augenposition dann erreicht werden, wenn mindestens zwei Infrarotstrahlungsquellen verwendet werden, deren Strahlungen jeweils unter einem Winkel von 30 bis 70°, bevorzugt zwischen 40 und 60°, zur optischen Achse der Behandlungs-Laserstrahlung verlaufen. Die optische Achse der Behandlungs-Laserstrahlung variiert zwar geringfügig beim Scannen, kann jedoch als im wesentlichen raumfest angesehen werden; insbesondere kann sie mit der optischen Achse des ruhenden Auges im wesentlichen gleichgesetzt werden.

Gemäß einer weiteren Variante der Erfindung sind für die Hilfsstrahlung zumindest drei Strahlungsquellen vorgesehen, deren Strahlungen jeweils unter einem Winkel von 30 bis 70°, bevorzugt zwischen 40 und 60°, zur optischen Achse der Behandlungsstrahlung verlaufen. Auch hierbei werden besonders bevorzugt Infrarot-Strahlungsquellen verwendet, bevorzugt in Form von lichtemittierenden Dioden (LED), insbesondere mit Wellenlängen von etwa 810 nm.

Die genannten Strahlungsquellen können selbst jeweils aus einer Vielzahl von LED zusammengesetzt sein. Eine "Strahlungsquelle" im Sinne dieser Anmeldung ist dann ein System mehrerer LED, die körperlich fest miteinander verbunden sind und deren zusammengesetzte Strahlung aus im wesentlichen derselben Richtung auf das Auge gerichtet wird.

Besonders bevorzugt ist vorgesehen, daß die Strahlungsquellen ein Dreieck bilden, unter dem das zu behandelnde Auge positionierbar ist. Das genannte Dreieck kann in einer Horizontalebene liegen oder in einer gegenüber der horizontalen leicht geneigten Ebene. Die Begriffe "horizontal" und "vertikal" werden hier im üblichen Sinn verwendet, d. h. in der Annahme, daß der liegende Patient horizontal ausgerichtet ist. Da elektromagnetische Strahlung für die hiesigen Zwecke als unabhängig von der Gravitation betrachtet werden kann und auch der Patient prinzipiell beliebig ausgerichtet werden kann, dienen die Begriffe "horizontal" und "vertikal" hier nur zur allgemeinen Erläuterung, wobei angenommen wird, daß der Patient in üblicher Weise liegt und sein Kopf mit dem Auge nach oben ausgerichtet ist.

Bei Verwendung von zumindest drei Strahlungsquellen für die Beleuchtungs-Hilfsstrahlung ist gemäß einer bevorzugten Ausgestaltung der Erfindung vorgesehen, daß die Strahlungen zumindest zweier Strahlungsquellen in einem spitzen Winkel relativ zueinander auf das zu behandelnde Auge zulaufen. Dieser spitze Winkel kann z. B. zwischen 25 und 70°, bevorzugt zwischen 35 und 65°, liegen. Bei einer bevorzugten Ausgestaltung dieser Variante der Erfindung ist vorgesehen, daß die Strahlung einer dritten Strahlungsquelle in Projektion auf einer Horizontalebene etwa in Richtung der Winkelhalbierenden des genannten spitzen Winkels zwischen den beiden anderen Strahlen verläuft.

Nachfolgend wird ein Ausführungsbeispiel der Erfindung anhand der Zeichnung näher erläutert. Es zeigt:
- Figur 1: schematisch einen vertikalen Schnitt durch ein zu behandelndes Auge und die Anordnung von mehreren Strahlungsquellen für die Hilfsstrahlung, und
- Figur 2: eine Draufsicht auf die Vorrichtung mit drei Strahlungsquellen für die Hilfsstrahlung.

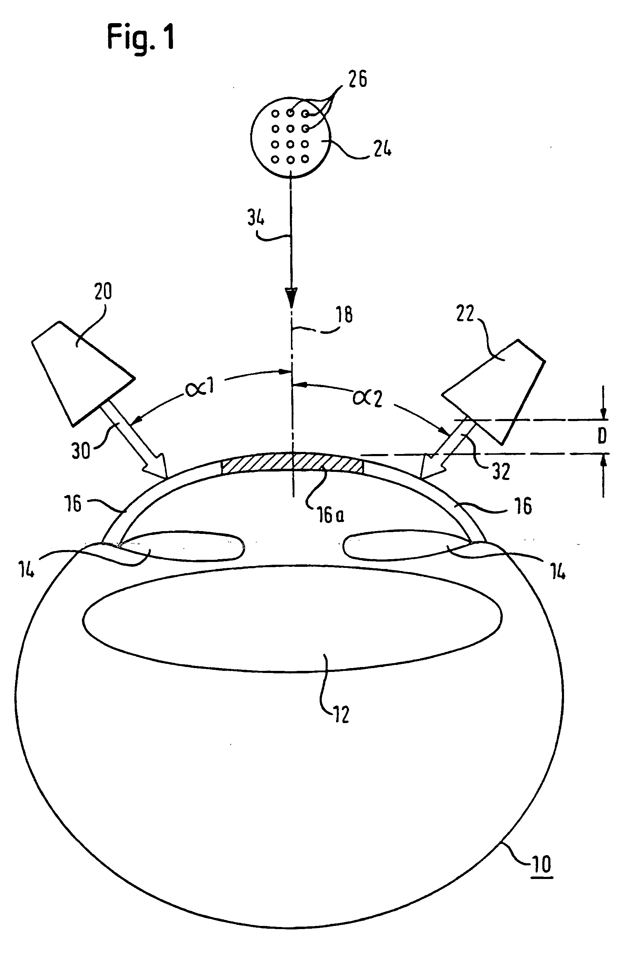

Figur 1 zeigt ein zu behandelndes Auge 10 mit einer Linse 12, einer Iris 14 und einer Cornea 16. Der mit Laserstrahlung (z.B. Excimer-Laserstrahlung mit einer Wellenlänge von 193 nm) zu behandelnde Bereich 16a der Cornea 16 ist in Figur 1 schraffiert.

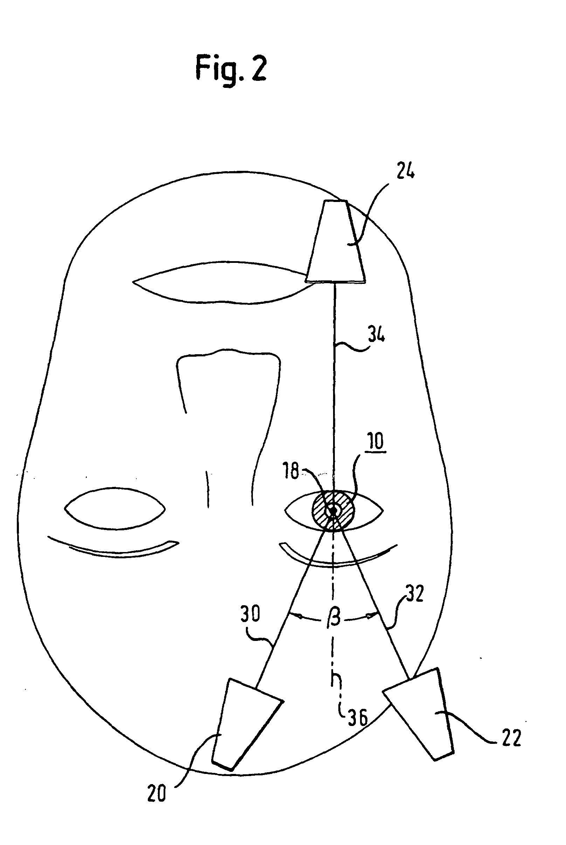

Figur 1 ist ein Vertikalschnitt durch das Auge 10. Figur 2 zeigt eine Draufsicht auf das zu behandelnde Auge 10 und die Anordnung von drei Strahlungsquellen 20, 22, 24 für die Hilfsstrahlung zur Ermittlung der Augenstellung. Das Kamerasystem und auch der eigentliche Behandlungs-Laserstrahl sind in den Figuren nicht gesondert dargestellt. Sie entsprechen dem oben genannten Stand der Technik. Die nachfolgende Beschreibung beschränkt sich deshalb auf die Hilfsstrahlung und deren Anordnung in Bezug auf das Auge.

Jede einzelne Strahlungsquelle 20, 22, 24 besteht aus einer Vielzahl von einzelnen lichtemittierenden Dioden (LED) 26. Die LED emittieren Strahlung im Infrarotbereich von 810 nm Wellenlänge. Für die Aufnahmekamera (nicht gezeigt) wird ein Tageslichtfilter verwendet. Es kann z. B. eine Schwarz-Weiß-Kamera verwendet werden. Zur Erzielung guter Meßergebnisse hinsichtlich der Augenstellung ist es wichtig, einen hohen Kontrast zwischen Pupille (dunkel) und Iris (hell) zu erreichen. Dies leistet die hier beschriebene Hilfsstrahlung der Strahlungsquellen 20, 22, 24.

Beim dargestellten Ausführungsbeispiel besteht jede Strahlungsquelle 20, 22, 24 aus zwölf einzelnen IR-LEDs, welche jeweils zu einem Array aus 3x4 LED verschaltet sind. Figur 1 zeigt eine der Strahlungsquellen (strahlungsquelle 24) so, daß die einzelnen LED 26 zu erkennen sind. Die beiden äußeren LED-Reihen aus jeweils vier LED haben z.B. einen Abstrahlwinkel von ± 8°, während die innere Reihe aus vier LED einen Abstrahlwinkel von ± 20° hat. Der genannte Abstrahlwinkel entspricht etwa dem Divergenzwinkel der Abstrahl.eule. Die genannte Wahl der Abstrahlwinkel ermöglicht eine Durchmischung der Strahlung innerhalb eines Arrays, was sich als vorteilhaft hinsichtlich einer schattenfreien und homogenen Ausleuchtung erwiesen hat. Eine unterschiedliche Wahl der Abstrahlwinkel innerhalb einer Strahlungsquelle wird bevorzugt bei allen drei Strahlungsquellen 20, 22, 24 eingesetzt.

Die aus den einzelnen LED jeweils erzeugten Strahlungen fügen sich jeweils zusammen zu einem zusammengefaßten Strahl 30, 32, 34. Die so entstehenden Strahlen sind in ihrer relativen Positionierung und in ihrer Positionierung im Bezug auf das zu behandelnde Auge 10 den Figuren 1 und 2 zu entnehmen.

In der Horizontaldarstellung gemäß Figur 2 verläuft der eigentliche Behandlungs-Laserstrahl (nicht gesondert gezeigt) senkrecht zur Zeichnungsebene; seine Achse ist mit 18 bezeichnet. Die Beleuchtungs-Strahlen (Hilfsstrahlen) 30, 32, 34 verlaufen leicht geneigt zur Horizontalebene und in einem Winkel α zur Achse 18 des Laser-Behandlungsstrahls. Figur 1 zeigt die Winkel zwischen den Beleuchtungsstrahlen 30, 32 und dem Behandlungs-Laserstrahl 18, nämlich die Winkel α₁ und α₂. Für die dritte Strahlungsquelle 24 gilt entsprechendes. Die Winkel α₁, α₂ liegen in einem Bereich von 40 bis 60°. Der Abstand D zwischen der Austrittsöffnung der Beleuchtungsstrahlen 30, 32, 34 und der Scheitelebene der zu behandelnden Cornea beträgt etwa 20 bis 150 mm, bevorzugt etwa 30 bis 100 mm.

Besonders vorteilhaft ist es, durch die beschriebene schräge Einstrahlung der Beleuchtungsstrahlung einen Lichteintritt des IR-Lichtes außerhalb der Behandlungszone 16a zu erreichen. Hierdurch wird sichergestellt, daß während der Behandlung keine wesentliche Änderung der Bedingungen für die Beleuchtungsstrahlung auftritt. Während der Behandlung kann sich die Transmission der Cornea innerhalb der Behandlungszone 16a drastisch ändern. Eine solche Änderung hat beim beschriebenen und in den Figuren 1 und 2 dargestellten Beleuchtungssystem keinen wesentlichen Einfluß auf die Beleuchtungsstrahlung und somit die Ermittlung der Augenstellung. Weiterhin werden störende, aber unvermeidliche Refelexe der IR-Beleuchtungsstrahlung an der Corneaoberfläche in den peripheren Bereich der Cornea verlagert. Hier führen sie zu keiner Störung der Auswertung der ermittelten Kontur der Pupille.

Die Figuren 1 und 2 lassen auch eine bevorzugte Anordnung der Strahlungsquellen relativ zueinander erkennen. Zwei Strahlungsquellen 20, 22 werden so angeordnet, daß die von ihnen emittierten Beleuchtungsstrahlen 30, 32 in Projektion auf eine Horizontalebene einen Winkel β bilden. Der Winkel β ist spitz, bevorzugt im Bereich von 25 bis 70°, besonders bevorzugt im Bereich von 35 bis 65°. Die dritte Strahlungsquelle 24 wird dann so angeordnet, daß ihre Strahlung 34 etwa im Bereich der Winkelhalbierenden 36 des Winkels β zwischen den beiden anderen Strahlen verläuft. Dies bewirkt eine optimale Ausleuchtung und beste Ergebnisse hinsichtlich der Ermittlung der Augenposition bei der Auswertung der Kamerabilder.

## Patentansprüche

1. Vorrichtung für eine medizinische Behandlung und/oder Operation des Auges mit Laserstrahlung unter Verwendung von Hilfsstrahlung zur Ermittlung der Augenstellung, wobei für die Hilfsstrahlung Infrarotstrahlungsquellen (20, 22, 24) vorgesehen sind, deren Strahlungen (30, 32, 34) unter einem Winkel α von 30 bis 70° zur optischen Achse (18) der Behandlungs- und/oder Operationsstrahlung verlaufen, **dadurch gekennzeichnet, dass** genau 3 Infrarotstrahlungsquellen (20, 22, 24) vorgesehen sind, die ein Dreieck derart bilden, dass die Strahlungen (30, 32) zweier Strahlungsquellen (20, 22) in einem spitzen Winkel (β) relativ zueinander auf das zu behandelnde Auge zulaufen und die Strahlung (34) der dritten Strahlungsquelle (24) in Projektion auf eine Horizontalebene in Richtung der Winkelhalbierenden (36) des spitzen Winkels (β) verläuft.

2. Vorrichtung nach Anspruch 1, **dadurch gekennzeichnet, dass** der Winkel (α) zwischen den Strahlungen (30, 32, 34) und der optischen Achse (18) zwischen 40° und 60° liegt.

## Claims

1. A device for a medical treatment and/or operation of the eye by means of laser radiation, with the utilisation of an auxiliary radiation for the determination of the position of the eyes, with infrared radiation sources (20, 22, 24) being provided for the auxiliary radiation, whose beams (30, 32, 34) extend under an angle (α) of 30° to 70° relative to the optical axis (18) of the treatment and/or operation radiation, **characterised in that** exactly 3 infrared radiation sources (20, 22, 24) are provided which form a triangle in such a manner that the beams (30, 32) of two radiation sources (20, 22) extend under an acute angle (β) relative to one another towards the eye to be treated, and the beam (34) of the third radiation source (24) in a projection onto a horizontal plane extends in the direction of the angle bisector (36) of the acute angle (β).

2. The device according to Claim 1, **characterised in that** the angle (α) between the beams (30, 32, 34) and the optical axis (18) ranges from 40° to 60°.

## Revendications

1. Dispositif pour un traitement médical et/ou une opération de l'oeil avec un rayonnement laser avec l'utilisation de rayonnement auxiliaire pour déterminer la position de l'oeil, des sources de rayonnement infrarouge (20, 22, 24), dont les rayonnements (30, 32, 34) sont agencés en formant un angle α de 30° jusqu'à 70° par rapport à l'axe (18) optique du rayonnement de traitement et/ou d'opération étant prévues pour le rayonnement auxiliaire, **caractérisé en ce qu'**exactement 3 sources de rayonnement infrarouge (20, 22, 24) sont prévues, qui forment un triangle de telle sorte que les rayonnements (30, 32) de deux sources de rayonnement (20, 22) arrivent sur l'oeil à traiter en formant un angle aigu (β) l'une par rapport à l'autre et le rayonnement (34) de la troisième source de rayonnement (24) est disposé en projection sur un plan horizontal en direction de la bissectrice (36) de l'angle aigu (β).

2. Dispositif selon la revendication 1, **caractérisé en ce que** l'angle (α) entre les rayonnements (30, 32, 34) et l'axe (18) optique se situe entre 40° et 60°.
